# EUROPEAN PATENT APPLICATION

(11) **EP 4 799 487 A1**
(43) Date of publication of application: **02.09.2026**
(21) Application number: 25160611.7
(22) Date of filing: 27.02.2025
(51) Int. Cl.: A23J 1/14, A23J 3/14, A23L 5/40, A23C 11/06, A23C 20/00, A23J 3/22, A61K 8/72

(54) **METHOD FOR LIGHTENING PEA PROTEIN ISOLATE**

(71) Applicant: KWS SAAT SE & Co. KGaA, 37574 Einbeck (DE)
(72) Inventor: Freter, Karl Frederik, 37574 Einbeck (DE)
(74) Representative: Eisenführ Speiser

(57) **Abstract**

The present invention is directed to methods for providing a plant-based protein ingredient having a consumer acceptable color, including vegetarian or vegan meat or dairy analogues. The protein ingredients have desired colors stemming from improved methodologies in the downstream processing of protein isolates.

## Description

### Technical Field

The present invention is directed to methods for providing a plant-based protein ingredient having a consumer acceptable color, including vegetarian or vegan meat or dairy analogues. The protein ingredients have desired colors stemming from improved methodologies in the downstream processing of protein isolates.

### Background

In view of an increasing consumer awareness regarding animal welfare and diet-associated illnesses, including obesity, type II diabetes, prevention of dementia and cardiovascular diseases, the development of healthier food products is of increasing interest and there is an ever-increasing consumer demand for plant-based food and food additives. In view of challenges related to climate change and a steady increase in the world population further increase the awareness related to ecological sustainability in the context of developing new seeds, planting and using the same.

Producers are thus facing the challenges of providing plant-based alternatives at a scale that meets consumers' expectations in taste, texture, and other functional and organoleptic properties, including color.

Color is one of the most important product-intrinsic sensory cues when it comes to setting people's expectations regarding the likely taste and flavor of food and drink. It was reported that a large body of laboratory research has demonstrated that changing the hue or intensity/saturation of the color of food and beverage items can exert a sometimes dramatic impact on the expectations, and hence on the subsequent experiences, of consumers (or participants in the lab). However, should the color not match the taste, then the result may well be a negatively valenced disconfirmation of expectation (Spence: On the psychological impact of food color. Flavour 4, 21 (2015). https://doi.org/10.1186/s13411-015-0031-3). As food color modulates the multisensory perception of flavor, as well as the consumer's appetitive and avoidance-related food behaviors, providing attractively colored food ingredients and food is thus of utmost importance when providing new plant-based food alternatives.

To meet the aforementioned demands of consumers, several sources of plant-based proteins have been considered by producers, including but not limited to: soybeans; peas, chickpeas and split peas; cranberries; beans such as navy, pinto, adzuki, fava, lima, black, red kidney, and mung beans; pumpkin seed and seed from other squash; and grains such as rice, sorghum, and millet. Consumers look for clean-labelled, locally-sourced and sustainable ingredients, which factors are also considered by producers. Further, certain consumers do not wish to consume animal products, including milk from cows, at all in view of its animal origin, due to lactose intolerance, or due to dairy allergies and the like. They may also see potential environmental sustainability issues. The global consumable industry has a continuing need to find plant-based protein sources that meet these requirements and preferences.

Peas (*Pisum sativum* or *Pisum sativum* L.) are annual plants with a one-year life cycle belonging to the family of *Leguminosae.* Peas belong to the oldest cultivated crops and domestication of wild pea plants likely already began with the start of agriculture about 11,000 years ago. Pea plants contain symbiotic bacteria within their root system with the special ability to fix nitrogen from the atmosphere, making them a highly attractive source of protein which meets the preferences of locally grown, sustainable agricultural practices.

One major challenge of working with pea plants, especially when it comes to the production of alternatives for milk etc., is the fact that to date, pea protein isolate as natural product suffers from severe batch-to-batch variations. Even if the same isolate is sourced from the same supplier there are massive differences in taste and particularly also color etc. This is due to the unknown potential of the underlying source: the pea.

Overcoming some of these bottlenecks e.g. masking in case of different off-flavor with aroma additives are possible, but not favorable due to elongated ingredient lists. However, in the case of color, the food industry reaches its limits when it comes to food applications, such as milk or poultry replacements, where a light to neutral coloring of the end-products is required, especially facing the fact that consumers usually inspect food and drink visually before deciding on whether or not to buy or taste it as relevant precondition for a successful and well-accepted food product, as deviations from the expected color (e.g., white for commercial milk products from animal sources) will trigger a deep-rooted warning system in human perception to avoid the consumption of "unexpected looking food" that might psychologically be qualified as potentially dangerous and thus less attractive.

It was thus a major object of the present invention to provide methods to be in a position to modify the pea plant ingredient's color in a targeted way to preferably provide protein isolates having a neutral-to-white color profile in a way that does not rely on chemicals for bleaching or energy-intensive steps.

### Brief Description of the Drawings

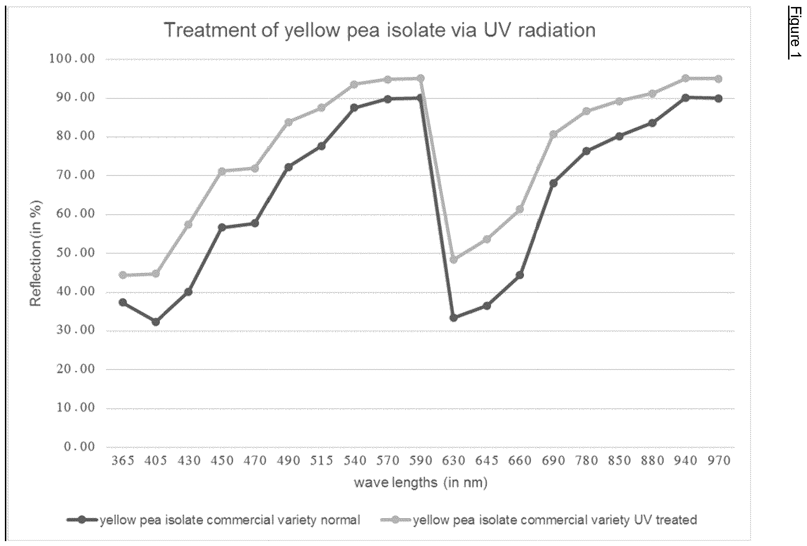

- **Figure 1:**: Graph of the VideometerLab measurement results of a pea protein isolate obtained from a yellow commercial line using either no or a UV treatment step. The black line shows the untreated values and the grey curve the values of a line treated with UV light.

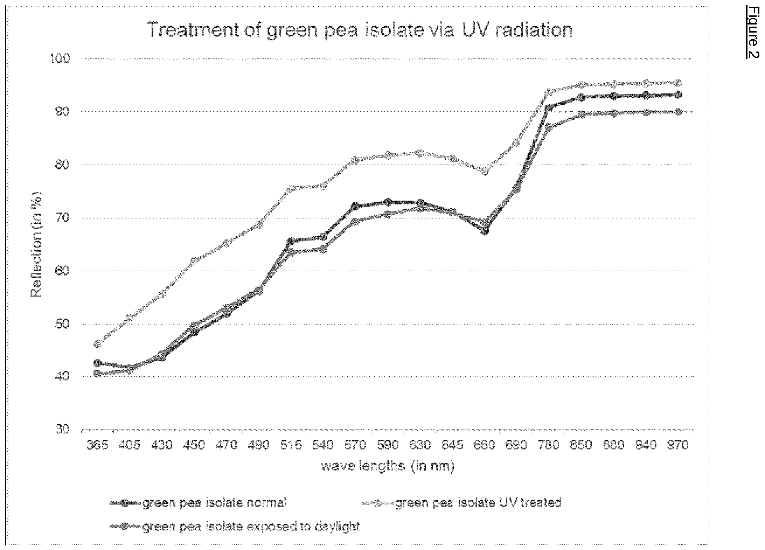

- **Figure 2:**: Graph of the VideometerLab measurements of a pea protein isolate obtained from a green pea line using different treatments. The dark grey curve shows the values for a non-treated pea protein isolate, the mid-grey curve shows the values of a pea protein isolate exposed to daylight and the light grey curve shows the values for a pea protein isolate treated with UV light. All tests were performed with the same pea line.

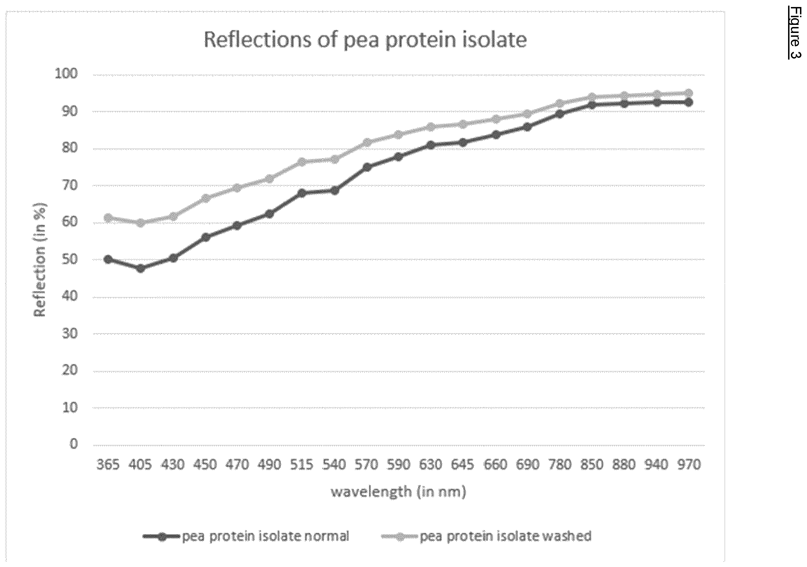

- **Figure 3:**: Graph of the VideometerLab measurements of a pea protein isolate, which was obtained using a washing step after obtaining the pea flour. The dark grey curve shows the values of a pea protein isolate obtained by a standard process and the light grey curve shows the reflection values of a pea protein isolate, which was obtained by a method including a washing step during protein isolation.

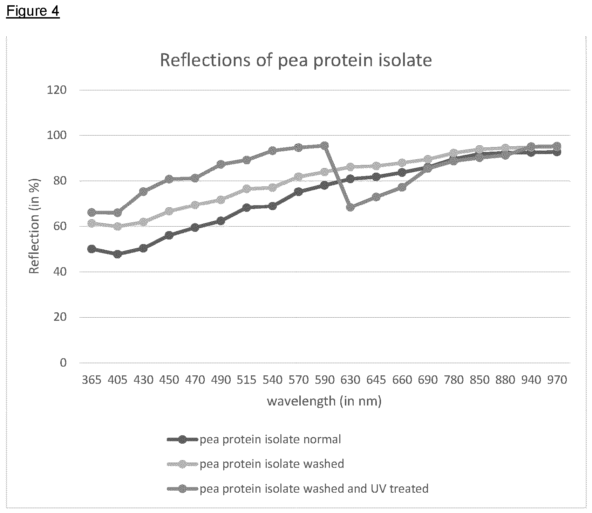

- **Figure 4:**: Graph of the VideometerLab measurements of a pea protein isolate, which was obtained using a washing step after obtaining the pea flour as well as a UV treatment step. The dark grey curve shows the reflection values of a pea protein isolate obtained by a standard process and the medium grey curve shows the reflection values of a pea protein ingredient, which was washed. The light grey curve shows the reflection values of a pea protein isolate, which was obtained by a method including a washing step as well as a UV treatment step during protein isolation.

### Definitions

An "alternative consumable product ingredient" as used herein refers to an ingredient from a plant, including a protein ingredient, which is suitable as part of a "consumable product" or of a "consumable composition". Further, at least a part or fraction of the "alternative consumable product ingredient" is suitable as a substitute for a commonly known and commonly fabricated product ingredient, usually it is suitable as an alternative to, for example, animal-derived or animal-produced ingredients or food, including meat, eggs or dairy.

An "alternative food" or "alternative nutrition" or "alternative (food) product / composition" as used herein refers to a food, including liquid food like beverages, which is usually a plant- or microorganism-based food that is an alternative to animal-derived food, including meat or dairy. Alternative food products may be of particular interest as an alternative source of proteins, but the term alternative food refers to any kind of nutritional building block, including proteins, carbohydrates, lipids, vitamins, minerals, fibers and the like that are suitable for food production and that are well accepted or even healthy as food and feed for human beings or farm animals and pets. An alternative food product or alternative food thus represents an "alternative consumable product" or an ingredient thereof. The ingredients of the present invention are useful to be implemented into an alternative food, an alternative feed or an alternative cosmetic.

An "alternative food product" as used herein may be present in liquid form, in semi-liquid form or in solid form. As used herein, an "alternative food product" refers to a product for human or animal consumption that is usually made with ingredients from animal sources, but in which the animal-sourced ingredient has been partially or fully replaced with a plant-based substitute ingredient. Non-limiting examples of alternative food products include alternative beverages such as a milk substitute or a drinkable yogurt substitute, or alternative food products such as an egg, beef, dairy, poultry, or seafood substitute. Other alternative food products include pet or animal feed products in which some or all of the animal-sourced ingredients are substituted with plant-based ingredients. The replacement or substitution of the animal-sourced ingredient may in some embodiments be e.g. more than 70%, more than 80%, more than 90%, or more than 95%. In other embodiments, the replacement or substitution of the animal-sourced ingredient may be 70% or less, such as 60%, 50%, 40%, etc. Alternative food products may also include non-dairy beverages such as sports drinks or smoothies. Alternative food products may further refer to alternative nutritional products, such as plant-based powder, to be used as dietary or nutritional supplements.

An alternative product or an alternative composition as used herein, and any pea protein ingredient suitable for the production thereof of the present invention is specifically processed (industrially and/or mechanically and/or chemically and/or enzymatically) and a pea protein ingredient of this invention will usually be processed, isolated, concentrated and/or otherwise treated for inclusion in an alternative product or composition. Additionally a pea protein ingredient of this invention will usually represent an intermediate ingredient, that was or that can be isolated from a plant representing one part or fraction of a final product or composition, or of a mixture or hybrid product. In an alternative product or composition, the pea protein ingredient of this invention as alternative part or fraction of the alternative product or composition thus substitutes a part or fraction that would be present in a commonly known and commonly fabricated product or composition, preferably, wherein it substitutes a part or fraction of animal or non-plant origin in the corresponding commonly known and commonly fabricated product.

Whenever the terms "composition" or "pea protein composition" is used herein, it refers to a composition of any of the protein ingredients, flours, concentrates or isolates disclosed herein that are used in an extracted form together with other ingredients from different origins to provide said composition. An alternative food or a cosmetic is thus also a composition in this sense. In this context, a "mixture" or "pea protein mixture" is a specific form of a composition, wherein pea protein fractions from different peas or even from different plants or other sources are mixed with each other to provide a basic protein mixture (and optionally further additives or ingredients of different nature and/or origin) comprising pea protein of the present invention.

A "consumable product" as used herein refers to goods that are usually understood to be used up or depleted during normal business operations, such as food and beverage, office supplies, cleaning and sanitary products, and medical supplies. In line with the general understanding, there are two main types of consumables: durable consumables, which are expected to last over a long period of time, and non-durable consumables, which are expected to be used up relatively quickly. Examples of consumables include perishable foods and beverages, paper products, ink cartridges, cleaning chemicals, gloves, and syringes. The consumable products particularly dealt with herein are non-durable consumables that are non-toxic when swallowed or applied on the human or animal body based on their intended use that are usually made of at least one organic raw materials (and optionally others), including food, beverages, gels, ointments, tooth paste and the like.

A "consumable product /composition" according to the present invention represents an alternative product or composition comprising at least one alternative plant-based ingredient, preferably a protein ingredient, according to the present invention, preferably a pea-based ingredient.

As used herein, "dairy substitute" or "dairy substitute composition" or "dairy alternative" or "dairy alternative product" refer to compositions that mimic the general appearance, nutritional content, and/or taste of dairy products produced using animal milk products without containing animal-based milk or being substantially free of animal-based products, and includes hybrid products made with lab-grown, fermented and animal-based components such as protein components. The dairy substitute may be completely free of any animal-based milk or animal-based milk protein or almost free of any animal-based milk protein, such as e.g. 90% free, or 95% free of any animal-based milk protein. The dairy substitute may be a dairy-free cheese, a dairy-free yogurt, a dairy-free ice cream, and the like.

Notably, the term "food" as used herein refers to a food intended for human nutrition, whereas a "feed" as used herein refers to a feed.

As used herein, "meat substitute" or "meat substitute composition" or "meat alternative" refers to compositions that mimic the general, organoleptic, and/or nutritional properties of consumable products produced using any type of meat or meat analog, including meat, fish, poultry, lab-grown and fermented meat products. This definition includes hybrid products made with lab-grown, fermented and animal-based components, such as protein components. A similar definition is used herein for "egg substitute", "egg substitute composition," and "egg alternative".

A "hybrid composition / product" or a "hybrid alternative (consumable, including food/cosmetic etc.) composition / product" as used herein refers to an alternative product that at least partially comprises an "alternative food" or "alternative nutrition" or "alternative (food) product" comprising a plant-originating protein substance according to the present invention, but which may comprise further ingredients.

The isoelectric point, also abbreviated as pl or IEP, is that pH at which a molecule, for instance a protein or a protein mixture, carries no net electrical charge, or is electrically neutral in the statistical mean, which is usually measured in solution for proteins. As it is known to the skilled person, the net charge on the molecule is affected by pH of its surrounding environment and can become more positively or negatively charged due to the gain or loss, respectively, of protons (H⁺).

A "(pea) protein flour" as used herein refers to an ingredient that contains milled peas.

"Protein texturate" or "textured protein" as used herein refers to an ingredient having a structural integrity and identifiable structure such that individual units, appearing as fibers, shreds, chunks, bits, granules, slices, and the like, will withstand hydration and cooking or other procedures used in the production of food for consumption. In general, textured proteins may be used to alter or enhance texture and bind water. Edible protein sources from which textured proteins are produced may include, but are not limited to, legumes (e.g., pulse protein), pea, soy, com, wheat, chickpea, potato, rice, sunflower, and the like. Textured proteins may include, but are not limited to, textured pea protein, textured soy flour, textured soy concentrate, textured wheat protein, textured potato protein, or combinations thereof. Methods for protein texturization are known and described in the art, and may include, for example, high temperature and pressure extrusion, spinning, freeze texturization, chemical or enzymatic texturization, and the like.

The terms *Pisum sativum* (L.) plant and pea plant, and short only pea, are used interchangeably herein, wherein the term pea is used in the context of the plant as a whole, but also to denote parts thereof, particularly seeds/fruits within pea pods.

A "pea plant ingredient" as used herein is to be understood as the total amount of protein that can be extracted from a pea fruit or seed (dry or fresh). A "(pea) protein ingredient" is in turn to be understood as the total amount of protein of the (pea) plant.

The term "protein concentrate" is a protein ingredient with a concentration of about 30% to 60%. A "protein isolate" is an even more concentrated protein ingredient with a concentration of about 60% to about 100%. A "protein flour" represent the protein that can be obtained directly after dehulling and milling. As this protein flour is not yet heavily processed, it reflects the original content of protein ingredients rather directly. Therefore, the "protein flour" was also used to define standard ratios by the inventors when comparing different material herein below. A "protein texturate" or "texturized vegetable protein" is used to describe a usually further defatted flour product that is particularly suitable and used as a meat analogue or meat extender. It is quick to cook, with a protein content comparable to some meats. The terms "protein flake" or "protein powder" further describe the form of the protein. A "protein powder" is usually composed of fine, dry particles produced by the grinding, crushing, or disintegration of a solid substance.

A "protein composition" as used herein refers to a protein isolate directly obtainable from a fruit, seed, particularly from a specific pea of the genus *Pisum,* or to a flour, a protein fraction, a purified or partially purified protein fraction. The protein composition, depending on the way of preparing the same, may include denatured and/or partially fragmented proteins, as the proteins may have undergone denaturation and/or fragmentation during thermal, chemical and/or mechanical processing/purification. A protein composition may consist of substantially one protein or a fragment thereof, or it may be a protein composition mixture comprising other proteins, particularly globulins and further pea proteins.

"UV light" as used herein refers to a type of electromagnetic radiation with wavelengths shorter than visible light but longer than X-rays, typically ranging from 10 nm to 400 nm. It is invisible to the human eye and is classified into three main types: UV-A (315-400 nm), UV-B (280-315 nm) and UV-C (100-280 nm). UV light is naturally emitted by the Sun and can also be produced artificially using UV lamps for applications like sterilization, fluorescence, and curing materials.

### Description of the Invention

The present invention is directed to improvement of the color of a protein isolate obtained from pea making the pea protein isolate highly attractive for alternative food and cosmetics production, especially for products and compositions, where a light color is desired and attracts customer' needs.

In one aspect, there is thus provided a method of providing a *Pisum sativum* protein isolate having a neutral-to-white color being defined by a reflection of more than 40% at a wavelength of 430 nm and a reflection of more than 75% at a wavelength of 590 nm, the method comprising the following steps:
i. providing at least one pea flour;
ii. isolating the protein ingredient from the pea flour;
   wherein at least one of the following steps is performed:
   (a) a washing procedure as part of the pea protein isolation step including the step of
      a.1 mixing the obtained pea flour from step i. with at least one buffer and adjusting the pH to the isoelectric point of the pea protein ingredient,
      a.2 removing the buffer; and
      a.3 drying the obtained mixture to yield a pea protein isolate; and/or
   (b) a UV treatment step, including the step of
      subjecting the pea protein flour of step i., the pea protein ingredient of step ii., and/or
      the pea protein isolate of step a.3. to exposure with UV light,
preferably wherein, only one UV treatment step is performed, more preferably, wherein the UV treatment step is performed on the provided pea flour from step i., on the provided pea protein isolate from step ii. or on the obtained pea protein isolate after the washing procedure obtained in step a.3. In terms of process efficiency, it is preferred that the UV treatment is performed on a pea protein isolate.

The isoelectric point of a molecule or a mixture of molecules can be determined by a suitable method known by the person skilled in the art. Such a suitable method can be e.g. the titration and parallel zeta potential measurement. The pH value of the solution at which the zeta potential is zero corresponds to the isoelectric point of the molecule or the mixture.

In one embodiment, step (b) is used as single UV treatment step, at least one UV treatment step, preferably exclusively one UV treatment steps is used, wherein the UV treatment step is usually performed on pea protein isolate.

In another embodiment, the at least one UV treatment is performed for a time individually selected from of between 1 minute to at least two hours, preferably 2 minutes to 1 hour, moreover preferably 4 to 40 minutes and especially preferably of 5 to 10 minutes. The length of the treatment steps can be varied. For example. In case more than one UV treatment step is used, the individual time of each step can be adapted accordingly to achieve a visually detectable reduction of the coloring.

In yet another embodiment, depending on the color of the pea raw material used, more than one UV treatment step can be used.

In yet another embodiment, provided that step (a) and (b) are both used, at least one UV treatment step is used, wherein the UV treatment step is usually directly performed with the obtained pea protein isolate after step a.3. Generally, the UV treatment step (b) can be performed before step (a) directly on the provided flour of step i, in case both steps are applied. In certain embodiments a UV treatment can be performed on the flour provided in step i. and at least one UV treatment step can be performed on the pea protein isolate after washing obtained in step a.3.

In certain embodiments, the UV treatment step (b) can be performed as single step, or as several UV treatment steps before or within or at the end of the washing procedure steps.

Notably, the present inventors surprisingly found that by including additional steps into conventional protocols for manufacturing pea protein isolate, the isolate itself has a brighter and lighter color and shows a neutral-to-white color. The discovered additional steps have an individual effect on the protein isolate color as such preferred for certain applications by the industry and consumer, or the resulting product shows an even better effect.

The color can be assessed by the reflection value of the pea protein isolate. This can be measured by various methods known in the art and at different wavelengths.

Step (i) of the method according to the present invention may include providing a commercially available pea protein flour or manufacturing a flour by dehulling and milling of the dried peas.

Step (ii) of the method according to the present invention may include isolating the protein ingredient by adding a base and thereby precipitation of the protein ingredient. Suitable methods for isolating the protein ingredient are known to the person skilled in the art.

In particular, the washing procedure (a) of the present invention is directly applied on the provided pea flour for aiding the isolation of the protein ingredient. The pH of the solution can be adjusted using sodium hydroxide or another suitable base.

The UV treatment (b) is applied on the obtained pea protein isolate, which can be either obtained with a method as described before or using the washing step according to the present invention.

In one embodiment of the above aspect, the reflection of the pea protein isolate is more than 45% at a wavelength of 430 nm and more than 80% at a wavelength of 590 nm, preferably more than 46% at a wavelength of 430 nm and more than 81% at a wavelength of 590 nm, especially preferably more than 47% at a wavelength of 430 nm and more than 83% at a wavelength of 590 nm, moreover preferably more than 48% at a wavelength of 430 nm and more than 82% at a wavelength of 590 nm.

The reflection is determined by measuring the amount of light at different wavelengths that is repelled from the sample. Suitable measurements for measuring light reflectance can be e.g. spectrometry, reflectometry or laser-based measurement techniques.

In yet another embodiment, there is provided a method, wherein the at least one buffer in step ii.1 is selected from water, ethanol, a solution of citric acid and its salts, phosphates, acetic acid and its salts and lactic acid and its salts.

In terms of the present invention, the use of water as buffer is particularly preferred.

In one embodiment of the methods as described herein, the buffer is removed by a technique selected from the group consisting of sedimentation, centrifugation and/or filtration.

In terms of the present invention, centrifugation is particularly preferred.

Yet another embodiment of the present invention relates to a method, wherein the UV light of step iii. has a wavelength of 100 to 280 nm.

In one embodiment of the present invention, the washing procedure (a) is repeated at least two times, at least three times, at least four times, at least five times, at least six times or at least seven times. It is also possible to repeat the washing step for more than seven times.

A second aspect of the present invention relates to a pea protein isolate, a pea protein texturate, a pea protein powder, or a pea protein flake of a *Pisum sativum* plant, having a neutral-to-white color being defined as a reflection of more than 40% at a wavelength of 430 nm and a reflection of more than 75% at a wavelength of 590 nm obtained or obtainable by a method according to the invention.

One embodiment of the second aspect of the present invention relates to a pea protein isolate, a pea protein texturate, a pea protein powder, or a pea protein flake, wherein the reflection of the pea protein isolate is more than 45% at a wavelength of 430 nm and more than 80% at a wavelength of 590 nm, preferably more than 46% at a wavelength of 430 nm and more than 81% at a wavelength of 590 nm, especially preferably more than 47% at a wavelength of 430 nm and more than 83% at a wavelength of 590 nm, moreover preferably more than 48% at a wavelength of 430 nm and more than 82% at a wavelength of 590 nm.

A third aspect of the present invention relates to a pea protein mixture or a pea protein composition comprising, a protein concentrate, a protein isolate, a pea protein texturate, a pea protein powder, or a pea protein flake of a *Pisum sativum* plant according to the invention and additionally comprising at least one further additive and/or ingredient.

The skilled person being a food chemist, expert, a chemist, or a process engineer will well be in a position to adjust the final content of a product to achieve the optimum conditions guaranteeing easy and efficient manufacturing as well as a hygienically safe, food- or cosmetics-grade product with the right degree of stability and non-perishable nature as needed.

A fourth aspect of the present invention relates to an alternative consumable or product, or a hybrid composition or product, being selected from a solid and a semi-solid alternative consumable or product, comprising at least one protein concentrate, a pea protein isolate, a pea protein texturate, a pea protein powder, or a pea protein flake of a *Pisum sativum* plant according to a second aspect of the invention, or comprising at least one mixture according to a third aspect of the invention, wherein the alternative consumable or an alternative cosmetic composition has an improved color in comparison to a reference material.

Such an alternative consumable or product may be a meat substitute product or a dairy alternative product. The meat products may include but are not limited to ground meat analogues, shredded meat analogues, and cut or fileted meat analogues. The dairy products may be hard, semi-hard, semi-soft, or soft cheese products, or spoonable or pourable dairy products, also including ice cream and the like.

In certain embodiments, the pea protein isolate, the pea protein texturate, the pea protein powder, or a pea protein flake of a *Pisum sativum* plant is incorporated into a milk alternative product.

According to certain embodiments disclosed herein, the alternative product is a hybrid product that comprises a pea protein ingredient of the present invention as well as at least one further additive and/or ingredient. Besides additives and ingredients typically used for food and cosmetic production, also at least one lab-grown material can be used. A lab-grown material as used herein refers to a cultured and/or fermented cell, and the material (any biomass, cellular and non-cellular, including supernatant) obtained therefrom, including bacterial cells, fungal cells, including filamentous fungi, particularly fungi and fungi biomass suitable as meat substitute, and cultured animal cells, including hepatocytes, myoblasts, osteoblasts, fibroblasts, lipoblasts, odontoblasts, adult neuronal progenitor cells, neural stem cells, multipotent stem cells from subventricular forebrain region, ependymal-derived neural stem cells, hematopoietic stem cells, liver-derived hematopoietic stem, marrow-derived stem cell, adipo-fibroblasts, adipose-derived stem cells, islet- cells producing stem cells, pancreatic-derived pluripotent islet-producing stem cells, mesenchymal stem cells, placenta cells, bone marrow stromal cells, muscle side population cells, bone marrow-derived recycling cells, blood-derived mesenchymal precursor cells, bone- marrow derived side population cells, muscle precursor cells, circulating skeleton stem cells, neural progenitor cells, multipotent adult progenitor cells, mesodermal progenitor cells, spinal cord progenitor cells and spore-like cell, and any combinations thereof, wherein the cell is not derived from a human embryo.

In certain embodiments, a consumable product, preferably an alternative food product or a hybrid product, as described herein may include one or more lipid composition(s), for example a fat, an oil, or combinations thereof. In general, fats refer to lipid compositions that are solid at room temperature, whereas oils are liquid at room temperature. The lipid compositions may include saturated fatty acids (also referred to as "saturated fats"), unsaturated fatty acids (also referred to as "unsaturated fats"), or combinations thereof. The lipid composition may include, but are not limited to, vegetable oil, coconut oil, palm oil, sunflower oil, soy oil, canola oil, or combinations thereof. The consumable product may include between 1% and 80%, between 1% and 70%, between 1% and 10%, between 1% and 5%, between 5% and 30%, between 10% and 25%, between 10% and 75%, or between 15% and 70% by weight of a lipid composition depending on the type of dairy substitute. An ordinarily skilled artisan will understand the appropriate lipid composition inclusion rate for a given composition.

The alternative food product, or the alternative cosmetic product, or a hybrid product, may include water as needed. For example, the product may include between 1% and 80%, between 5% and 75%, between 15% and 70%, between 45% and 65%, between 50% and 60%, between 1% and 20%, or between 5% and 15% by weight of water depending on the type of consumable product.

In some embodiments, the consumable product comprising a pea protein ingredient, flour, protein concentrate, protein isolate, pea protein texturate, pea protein powder, or a pea protein flake of a *Pisum sativum* plant, having a neutral-to-white color being defined by a reflection of more than 40% at a wavelength of 430 nm and a reflection of more than 75% at a wavelength of 590 nm includes both water and a plant-based ingredient. The total of the water and plant-based ingredient may be e.g. between 50% and 95% or between 60% and 90% by weight of the composition.

In certain embodiments, the consumable product comprising a pea protein ingredient, flour, protein concentrate, protein isolate, pea protein texturate, pea protein powder, or a pea protein flake of a *Pisum sativum* plant, having a neutral-to-white color being defined by a reflection of more than 40% at a wavelength of 430 nm and a reflection of more than 75% at a wavelength of 590 nm may include fiber. The fiber may include, but is not limited to, pectin, apple fiber, psyllium, flax fiber, rice bran extract, Konjac flour, and the like. The consumable product may include between 0.01% (wt) and 3% (wt), between 0.05% (wt) and 2% (wt), or between 0.1% (wt) and 2% (wt) of fiber. The consumable product may include fiber in an amount up to 0.5% (wt), up to 1% (wt), up to 1.5% (wt), up to 2% (wt), up to 2.5% (wt), or up to 3% (wt).

In certain embodiments, the consumable product comprising a pea protein ingredient, flour, protein concentrate, protein isolate, pea protein texturate, pea protein powder, or a pea protein flake of a *Pisum sativum* plant, having a neutral-to-white color being defined by a reflection of more than 40% at a wavelength of 430 nm and a reflection of more than 75% at a wavelength of 590 nm may include starch. The starch may include a pregelatinized starch, a modified starch, or combinations thereof. The starch may include, but is not limited to, com starch, potato starch, tapioca starch, and the like. The consumable product may include between 0.5% (wt) and 25% (wt), between 1.0% (wt) and 20% (wt), or between 2% (wt) and 18% (wt) of starch. The consumable product may include a hydrocolloid. For example, the consumable product may include guar gum, xanthan gum, locust bean gum, carrageenan, cellulose, konjac gum, and combinations thereof. The consumable product may include between 0.01% and 5%, between 0.05% and 4.5%, between 0.1% and 4.0%, or between 0.5% and 3.8% by weight of hydrocolloid. The consumable product may include up to 5%, up to 4.5%, up to 4.0%, up to 3.8%, up to 3.5%, up to 2.5%, up to 2.0%, or up to 1.0% by weight of hydrocolloid.

In some embodiments, the consumable product comprising a pea protein ingredient, flour, protein concentrate, protein isolate, pea protein texturate, pea protein powder, or a pea protein flake of a *Pisum sativum* plant, having a neutral-to-white color being defined by a reflection of more than 40% at a wavelength of 430 nm and a reflection of more than 75% at a wavelength of 590 nm may include lecithin. The consumable product may include between 0.01% and 10%, between 0.05% and 8.0%, or between 0.1% and 5% by weight lecithin.

In certain embodiments, the consumable product comprising a pea protein ingredient, flour, protein concentrate, protein isolate, pea protein texturate, pea protein powder, or a pea protein flake of a *Pisum sativum* plant having a neutral-to-white color being defined by a reflection of more than 40% at a wavelength of 430 nm and a reflection of more than 75% at a wavelength of 590 nm may include a preservative. For example, the consumable product may include a preservative such as, but not limited to potassium sorbate. The consumable product may include a preservative in an amount up to 0.1%, up to 0.5%, or up to 1.0% by weight of the consumable product. The consumable product may include a flavor or seasoning. For example, the consumable product may include a natural or artificial flavor(s) and/or seasonings. Seasonings may include, but are not limited to, sweetener(s), salt (e.g., sodium chloride, potassium chloride, and the like), cocoa, chocolate, cinnamon, nutmeg, coconut, almond, combinations thereof, and the like. The consumable product may include between 1% and 20%, between 1.5% and 10%, between 5% and 20%, or between 2% and 18% of a flavor or seasoning. The consumable product may be free of any flavors or seasoning. In some embodiments, the consumable product may include between 0.001% and 3.0%, between 0.01% and 2.0%, or between 0.025% and 1.75% of a salt. The consumable product may be free of salt.

In yet another embodiment, the consumable product comprising a pea protein ingredient, flour, protein concentrate, protein isolate, pea protein texturate, pea protein powder, or a pea protein flake of a *Pisum sativum* plant, having a neutral-to-white color being defined by a reflection of more than 40% at a wavelength of 430 nm and a reflection of more than 75% at a wavelength of 590 nm may include a sweetener. Suitable sweeteners are known and described in the art. The sweetener can be at least one of a non-caloric sweetener or a caloric sweetener. The sweetener can be any type of sweetener, for example, a sweetener obtained from a plant or plant product, or a physically or chemically modified sweetener obtained from a plant, or a synthetic sweetener. Exemplary sweeteners include steviol glycosides, mogrosides, sucrose, fructose, glucose, erythritol, maltitol, lactitol, sorbitol, mannitol, xylitol, tagatose, trehalose, galactose, rhamnose, cyclodextrin (e.g., a-cyclodextrin, b-cyclodextrin, and g-cyclodextrin), ribulose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, glucosamine, mannosamine, fucose, fuculose, glucuronic acid, gluconic acid, glucono-lactone, abequose, galactosamine, xylo-obgosaccharides (xylotriose, xylobiose and the like), gentio- obgoscaccharides (gentiobiose, gentiotriose, gentiotetraose and the like), galacto- obgosaccharides, sorbose, ketotriose (dehydroxyacetone), aldotriose (glyceraldehyde), nigero- obgosaccharides, fructoobgosaccharides (kestose, nystose and the like), maltotetraose, maltotriol, tetrasaccharides, mannan-oligosaccharides, maltooligosaccharides (maltotriose, maltotetraose, maltopentaose, maltohexaose, maltoheptaose and the like), dextrins, lactulose, melibiose, raffmose, rhamnose, ribose, sucralose, acesulfame K, aspartame, saccharin, coupling sugars, soybean oligosaccharides, and combinations thereof. D- or L-configurations can be used when applicable.

The consumable product comprising a pea protein ingredient, flour, protein concentrate, protein isolate, pea protein texturate, pea protein powder, or a pea protein flake of a *Pisum sativum* plant, having a neutral-to-white color being defined by a reflection of more than 40% at a wavelength of 430 nm and a reflection of more than 75% at a wavelength of 590 nm may include an acid. Suitable acids include, but are not limited to, citric acid, lactic acid, sorbic acid, malic acid, combinations thereof, and the like. The consumable product may include an acid in an amount up to 0.001%, up to 0.005%, up to 0.01%, up to 0.1%, up to 1.0%, up to 1.5%, or up to 2.0% of the consumable product. The consumable product may include between 0.0001% and 2.0%, between .0002% and 1.5%, between 0.0003% and 1.0% by weight of an acid.

In certain embodiments, a hybrid food or composition may thus comprise at least one portion being a pea protein ingredient, protein concentrate, protein isolate, pea protein texturate, pea protein powder, or a pea protein flake of a *Pisum sativum* plant of the present invention and at least one portion being a lab-grown material, preferably wherein the lab-grown material stems from a bacterial fermentation, or from a fungus, preferably from a filamentous fungus. Filamentous fungal biomass, usually named mycoproteins, is a suitable meat substitute since it is nutritious and has filaments and thus a texture perfectly mimicking meat fibrils. Regarding the type of residual water, nutrient supplementation, optimum conditions for biomass production, and characteristics of the mycoproteins, the optimum growth condition can be at about pH of 4.5. Mycoprotein usually contains 19.44% (*wt.*/*wt.*) protein with a high crude fiber content of 8.51% *(wt.*/*wt.)* and a low fat content of 1.56% *(w*/*w).* In addition, the amino acid and fatty acid contents are dominated by glutamic acid and polyunsaturated fatty acids, which are associated with an umami taste (Wikandari et al., 2023, https://doi.org/10.3390/molecules28030997). Particularly the physicochemical and the viscoelastic properties of mycoproteins and the pea protein ingredients of the present invention allow that both sources of alternative proteins are easily compounded and used together for food, particularly alternative meat or fish design, as the pl values of the major protein fractions and the viscoelastic properties are perfectly suitable to produce food with a high nutrition value and an excellent texture and stability.

In one embodiment of the fourth aspect, the alternative food or the cosmetic composition or product, or the hybrid composition or product thereof has a pH in the range of about 3.5 to about 8.5, preferably a pH from about 4.5 to about 8.

In a fifth aspect of the present invention, a use of a pea protein concentrate, a protein isolate, a pea protein texturate, a pea protein powder, or a pea protein flake of a *Pisum sativum* plant as defined herein, or a use of a mixture as defined herein for preparing an alternative consumable composition or an alternative cosmetic composition, preferably wherein the consumable composition is a solid or semi-solid alternative consumable composition or an alternative cosmetic composition.

While several possible aspects are disclosed above, embodiments of the present invention are not so limited. These exemplary aspects are not intended to be exhaustive or to unnecessarily limit the scope of the invention, but instead were chosen and described in order to explain the principles of the present invention so that others skilled in the art may practice the invention. Indeed, various modifications of the invention in addition to those described herein will become apparent to those skilled in the art from the foregoing description. Such modifications are intended to fall within the scope of the appended claims.

The present invention is, in particular, captured by any one or any combination of one or more of the above-mentioned aspects, with any other statement and/or embodiments.

The following Examples serve to provide further details on embodiments and enablement of the invention, but do not necessarily limit the scope of the invention.

### Examples

### Example 1: Production of pea flour from whole peas

The material used was as follows: whole peas, any suitable variety, for example the variety Proklam, several kilograms; several bowls of adequate size for sorting; scoops and a brush; a Streckel&Schrader laboratory dehuller LU-200; a Streckel&Schrader laboratory airleg separator LST1 and a ultracentrifugal mill Retsch ZM200 with cyclone, attached bowl and 0.2 mm sieve.

To obtain a pea protein flour, the following sample preparation was conducted: for the dehulling step, the whole peas were put into the dosing funnel of the dehuller. Using the dosing channel, the speed and amount of peas falling into the dehulling chamber can be adjusted. The slit between the dehuller discs can be adjusted to the size of the peas. The mix of dehulled whole and split peas and their hulls is collected in a drawer below the discs and is transferred into the dosing funnel of the airleg separator in order to sort them apart. The air flow to the different chambers therein can be adjusted with two screws. Peas that are still not dehulled, are transferred back to the dehuller while adjusting the slit accordingly.

Dehulled peas were then milled with the ultracentrifugal mill and together with the mentioned components, carefully dosing them with a scoop. The resulting flour is collected in a bowl, and residues on parts of the mill are brushed off in order to clean them.

### Example 2: Extraction of Pea Protein Isolate (PPI) from pea flour

To this end, the following material was used: pea flour (e.g., as produced in Example 1 above), 1M NaOH, 6 M HCl, distilled water, 3 L and 5 L beakers, a lab scale PCE Instruments PCE-BSH 10000, a propeller stirrer VWR VOS 40 digital, a magnetic stirrer IKA RH 2 basic, a centrifuge Eppendorf 5910 Ri cooled, with 4 centrifuge bottles 1L, a disperser/ homogenizer IKA ultraturrax T50 digital with S50N G45G disperser tool, a pH meter Mettler Toledo FiveEasy F20, a spoon, silicone spatula and metal spatula, and a spray dryer Büchi S-300.

Pea flour was dispersed into distilled water while stirring with a propeller stirrer at around 400 *rpm* in a ratio of S:L of 1:7, e.g. 600 g flour and 3000 mL water. The pH value of the dispersion was measured and adjusted using a pH-meter and 1M NaOH until it reached pH 8. Extraction was conducted at 150 *rpm* at room temperature (at about 20 to 22°C) for 1 h. The dispersion was then transferred into the centrifugation bottles and centrifuged at 4347 x g for 25 *min* to separate the dissolved protein-rich supernatant from the pellet rich in starch and fibers. The pellet was disposed. The supernatant was transferred to the 3L beaker and stirred with the magnetic stirrer in order at 500 *rpm* to carefully dose the 6M HCL to precipitate the protein. The pH was adjusted to the isoelectric point of the proteins at 4.5, and the protein dispersion was stirred for 10 *min* to help the proteins agglomerate better. The dispersion was then centrifuged at 4347 x g for 25 *min* at 7°C to lower the protein solubility further and improve the protein yield. The resulting supernatant was disposed, and the protein pellet was carefully removed from the bottles with spatulas and transferred into a beaker. The protein was washed with distilled water at a ratio of S:L of 1:5, using a ultraturrax disperser. The washed protein was again centrifuged under aforementioned conditions, and collected in a beaker. Neutralisation was carried out using the ultraturrax homogeniser, distilled water, until a protein concentration of about 10% was reached, and 1M NaOH. The neutralized protein solution was then spray-dried under the following conditions: inlet temperature 130°C, spraying gas flow 900 L/h, drying gas flow 35 m³, product flow 6 mL/min. With these adjustable parameters the resulting outlet temperature was kept between 70-75 °C to ensure a safe product while maintaining the functionalities of native proteins as much as possible.

Specific parameters of this process are not intended to be limiting, and variations of this extraction process by adjusting the pH, temperature and standing time, and other parameters may be utilized.

### Example 3: Measuring of pea protein isolate color

Pea protein was obtained according to Example 1 and Example 2 from three different lines, wherein Bulk is a commercially available pea line, KWS1 and KWS2 are pea lines according to the invention.

The reflection of each of the isolates was examined using VideometerLab. Analysis of the obtained data was done using the VideometerLab software.

Firstly, 1 g of pea protein isolate of each of the tested lines was placed in a Petri dish. The isolate was then compressed with the spatula to create an even surface. This even surface was necessary to ensure a reliable color measurement. Afterwards, the Videometerlab measured the reflection of the sample placed in the sample chamber at the following wavelengths (all values in nm): 365, 405, 470, 515, 540, 590, 645, 780, 850, 970, 430, 450, 490, 570, 630, 660, 690, 880 and 940.

The results of this measurement are also shown in Figure 1 to 3 for pea protein isolate treated with UV light or with a washing step. It can be observed that either the washing step or the UV treatment step results in a higher reflection of the pea protein isolate, which equally correlates to a lighter color.

### Example 4: Treatment of the pea protein isolate with washing

The peas were dehulled and processed according to Example 1. After obtaining a pea flor, the extract was washed. By shifting the pH to the isoelectric point of the protein, in this case 4.5, the protein precipitates while colored components remain in solution. This supernatant is discarded, and the protein pellet is further processed.

The pea flour was transferred to a suitable beaker and mixed on a magnetic stirrer at level 5. To wash the proteins, the pH was adjusted to 4.5 with 6 M HCl. This state was maintained for a 5 to 10 minutes, and then the solution was centrifuged at 7°C, 4500 rpm for 25 minutes in the precipitation program. The supernatant was discarded, and the pellet further processed. This washing step was applied two times.

This solution was transferred to the spray dryer via a peristaltic pump. The spray dryer was operated with the following settings:
- Drying gas: 35 m³/h
- Inlet temperature: 135°C
- Spray gas: 900 L/min
- Pump 1: 6 mL/min
- Outlet temperature: 90°C (ideal at 75°C)
- Unclogging: 5 bpm
- Filter pressure: 1 mbar

The resulting isolate removed from the spray dryer and stored in labeled vacuum bags.

The resulting pea protein isolate was then subjected to reflection measurement using a VideometerLab according to Example 3. The results of this measurement are shown in Figure 3.

### Example 5: Treatment of the pea protein isolate using UV exposure

The pea protein isolate was manufactured according to Example 1 using pea material of a commercial pea line and was further processed using UV light exposure. For this, 2 g of the pea protein isolate were weighed into a petri dish and the petri dish placed under a UV lamp. The pea protein isolate was then exposed to UV light for five minutes and after that, the pea protein isolate is stirred until the desired color is achieved. Afterwards, the reflection and thus color of the pea protein isolate was measured using VideometerLab according to Example 3.

The obtained values for the reflection are shown in Figures 1 and 2. For comparison, pea protein isolate that was exposed for two months to daylight was measured. It can be observed that the reflection of the pea protein isolate that was exposed to UV light is higher compared to the pea protein isolate, which was only exposed to daylight.

### Example 6: Treatment of the pea protein isolate using UV exposure and washing

The peas were dehulled and processed according to Example 1. After obtaining a pea flor, the extract was washed. By shifting the pH to the isoelectric point of the protein, in this case 4.5, the protein precipitates while colored components remain in solution. This supernatant is discarded, and the protein pellet is further treated with UV light.

For this, 2 g of the pea protein isolate were weighed into a petri dish and the petri dish placed under a UV lamp. The pea protein isolate was then exposed to UV light for five minutes and after that, the pea protein isolate is stirred until the desired color is achieved. Afterwards, the reflection and thus color of the pea protein isolate was measured using VideometerLab according to Example 3.

The obtained values for the reflection is shown in Figure 4. For comparison, untreated pea protein isolate as well as washed pea protein isolate was measured. It can be observed that the reflection of the pea protein isolate that was washed and exposed to UV light is higher compared to the pea protein isolate, which was only washed.

## Claims

1. A method of providing a pea protein concentrate, a protein isolate, a pea protein texturate, a pea protein powder, or a pea protein flake of a *Pisum sativum* plant having a neutral-to-white color being defined by a reflection of more than 40% at a wavelength of 430 nm and a reflection of more than 75% at a wavelength of 590 nm, the method comprising the following steps:
i. providing at least one pea flour;
ii. isolating the protein ingredient from the pea flour;
wherein at least one of the following steps is performed:
(a) a washing procedure as part of the pea protein isolation step including the step of:
a.1 mixing the obtained pea flour from step i. with at least one buffer and adjusting the pH to the isoelectric point of the pea protein ingredient,
a.2 removing the buffer; and
a.3 drying the obtained mixture to yield a pea protein isolate;
and/or
(b) a UV treatment step, including the step of subjecting the pea protein flour of step i., the pea protein ingredient of step ii., and/or the pea protein isolate of step a.3. to exposure with UV light,
preferably wherein, only one UV treatment step is performed, more preferably, wherein the UV treatment step is performed on the provided pea flour from step i., on the provided pea protein isolate from step ii. or on the obtained pea protein isolate after the washing procedure obtained in step a.3.

2. The method of claim 1, wherein the reflection of the protein concentrate, the protein isolate, the pea protein texturate, the pea protein powder, or the pea protein flake is more than 45% at a wavelength of 430 nm and more than 80% at a wavelength of 590 nm, preferably more than 46% at a wavelength of 430 nm and more than 81% at a wavelength of 590 nm, especially preferably more than 47% at a wavelength of 430 nm and more than 83% at a wavelength of 590 nm, moreover preferably more than 48% at a wavelength of 430 nm and more than 82% at a wavelength of 590 nm.

3. The method according to claim 1 or 2, wherein the at least one buffer in step ii.1 is selected from water, ethanol, a solution of citric acid and its salts, phosphates, acetic acid and its salts as well as lactic acid and its salts, or mixtures thereof.

4. The method according to any one of the preceding claims, wherein the buffer is removed by a technique selected from the group consisting of sedimentation, centrifugation and/or filtration.

5. The method according to any of the preceding claims, wherein the UV light has a wavelength of 100 to 280 nm.

6. The method according to any one of the preceding claims, wherein the washing procedure (a) is repeated at least two times
and/or
the UV treatment step (b) is performed for a time individually selected from of between 1 minute to at least two hours, preferably of between 2 minutes to 1 hour, moreover preferably of between 4 to 40 minutes and especially preferably of between 5 to 10 minutes.

7. A pea protein isolate, a pea protein texturate, a pea protein powder, or a pea protein flake of a *Pisum sativum* plant, having a neutral-to-white color being defined by a reflection of more than 40% at a wavelength of 430 nm and a reflection of more than 75% at a wavelength of 590 nm, obtained or obtainable by a method according to any one of claims 1 to 6.

8. The pea protein isolate according to claim 7, wherein the reflection of the pea protein isolate, the pea protein texturate, the pea protein powder, or the pea protein flake is more than 45% at a wavelength of 430 nm and more than 80% at a wavelength of 590 nm, preferably more than 46% at a wavelength of 430 nm and more than 81% at a wavelength of 590 nm, especially preferably more than 47% at a wavelength of 430 nm and more than 83% at a wavelength of 590 nm, moreover preferably more than 48% at a wavelength of 430 nm and more than 82% at a wavelength of 590 nm.

9. A pea protein mixture or a pea protein composition comprising, a pea protein concentrate, a pea protein isolate, a pea protein texturate, a pea protein powder, or a pea protein flake of a *Pisum sativum* plant and additionally comprising at least one further additive and/or ingredient.

10. An alternative consumable or product, or a hybrid composition or product, being selected from a solid and a semi-solid alternative consumable or product, comprising at least one pea protein concentrate, a pea protein isolate, a pea protein texturate, a pea protein powder, or a pea protein flake of a *Pisum sativum* according to claim 7 or 8, or comprising at least one mixture of claim 9, wherein the alternative consumable or an alternative cosmetic composition has an improved color in comparison to a reference material.

11. The alternative consumable or the cosmetic composition or product, or the hybrid composition or product thereof of claim 10, wherein the composition or product has a pH in the range of about 3.5 to about 8.5, preferably a pH from about 4.5 to about 8.

12. A use of a protein concentrate, a protein isolate, a pea protein texturate, a pea protein powder, or a pea protein flake of a *Pisum sativum* plant as defined in claim 7 or 8, or a use of a mixture as defined in claim 9 for preparing an alternative consumable composition or an alternative cosmetic composition, preferably wherein the consumable composition is a solid or semi-solid alternative consumable composition or an alternative cosmetic composition.
